(19) Europäisches Patentamt
European Patent Office
Office européen des brevets

(11) **EP 3 284 405 B1**

(12) **EUROPÄISCHE PATENTSCHRIFT**

(45) Veröffentlichungstag und Bekanntmachung des Hinweises auf die Patenterteilung:
**08.04.2020 Patentblatt 2020/15**

(51) Int Cl.:
***A61B 5/087*** *(2006.01)*

(21) Anmeldenummer: **17185670.1**

(22) Anmeldetag: **10.08.2017**

(54) **MESSGERÄT UND VERFAHREN ZUM BESTIMMEN EINER MAXIMALEN INHALATIONSLEISTUNG EINER PERSON**

MEASURING APPARATUS AND METHOD FOR DETERMINING THE MAXIMUM INHALATION OF A PERSON

APPAREIL DE MESURE ET PROCÉDÉ DE DÉTERMINATION D'UNE CAPACITÉ D'INHALATION MAXIMALE D'UN SUJET

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.08.2016 DE 102016115074**

(43) Veröffentlichungstag der Anmeldung:
**21.02.2018 Patentblatt 2018/08**

(73) Patentinhaber: **Fraunhofer-Gesellschaft zur Förderung der angewandten Forschung e.V.
80686 München (DE)**

(72) Erfinder:
• **Pohlmann, Gerhard
31715 Meerbeck (DE)**
• **Ramazanoglu, Mehmet
30622 Hannover (DE)**

(74) Vertreter: **Raffay & Fleck
Patentanwälte
Grosse Bleichen 8
20354 Hamburg (DE)**

(56) Entgegenhaltungen:
**EP-A1- 0 937 438        US-A- 5 749 368
US-A1- 2011 124 470        US-A1- 2014 283 831
US-A1- 2015 258 370**

**Beschreibung**

Die vorliegende Erfindung betrifft ein Messgerät und ein Verfahren zum Bestimmen einer maximalen Inhalationsleistung einer Person.

Bei der Anwendung von Trockenpulverinhalatoren für die Verabreichung von Trockenpulverinhalat an eine Person, einen Patienten, muss der Patient in der Regel so tief und kräftig als möglich einatmen. Denn nur so kann eine Inhalation mit einem schnellen Anstieg des Volumenstroms am Beginn des Verabreichungsintervalls des Trockenpulverinhalats erreicht werden. Dieser Anstieg des Volumenstroms ist zu dem bezeichneten Zeitpunkt erforderlich, um die für eine ausreichende Dispergierung der Medikamentenformulierung erforderliche Energie aufzubringen. Hierbei gilt: Je besser die Dispergierung, umso besser ist in der Regel auch die Qualität der von dem Trockenpulverinhalator emittierten Dosis in Bezug auf die Partikelgröße der Trockenpulverpartikel.

Verschiedene Trockenpulverinhalatoren haben dabei unterschiedliche Atemwiderstände; unterschiedliche Patienten haben unterschiedliches Atemvermögen, also erreichbare Spitzenatemleistungen im Hinblick auf z.B. Volumenstrom oder Strömungsgeschwindigkeit des Atemstroms. Die von Patienten erreichbare Spitzenatemleistung ist häufig bei Trockenpulverinhalatoren mit geringem Atemwiderstand höher, was in verstärktem Ausmaß auch für die Strömungsgeschwindigkeit des durchsaugten Atemvolumens gilt. Andererseits darf aber die Strömungsgeschwindigkeit bei Eintritt der mit Trockenpulverinhalat versetzten Atemluft in den Rachenraum des Patienten nicht zu hoch sein. Denn ansonsten besteht die Gefahr einer vorzeitigen Abscheidung des Trockenpulverinhalats, das jedoch in tieferen Zonen des Atemapparats, in den Bronchien und bis hinein in die tiefen Lungenbereiche, wirken und erst dort adsorbiert werden soll. Insoweit ist es von erheblicher Bedeutung, für jeden Patienten anhand seines individuellen Atemvermögens den passenden Trockenpulverinhalator auszuwählen.

Somit muss also zunächst ein Weg gefunden werden, das Atemvermögen eines jeden Patienten, welches sich insbesondere durch die Leistung in der Einatemspitze, also die maximale Leistung bei der Inspiration, und die korrespondierende Einatemgeschwindigkeit charakterisieren lässt, individuell zu bestimmen. Diese Bestimmung sollte dabei schnell und kostengünstig sowie zuverlässig unternommen werden können.

Aus der EP 937 438 A1 ist ein Inhalationstestgerät bekannt, das stabförmig aufgebaut ist. Es weist ein Mundstück auf, das mit einem Luftkanal verbunden ist. Strömungstechnisch vor dem Mundstück ist ein Strömungswiderstand in Form einer Blendenöffnung angeordnet. Jenseits der Blendenöffnung und in Strömungsrichtung beim Einatmen gesehen vor diese geschaltet ist ein Zylinder mit einem darin angeordnetem, federbelasteten Kolben. An seinem dem Mundstück abgewandten Ende weist der Zylinder Lufteinströmöffnungen auf. Atmet eine Person durch dieses Gerät Luft ein, so wird der Kolben in dem Zylinder verschoben und zeigt den beim Atemvorgang erreichten maximalen Volumenstrom pro Zeiteinheit an. In der US 4,693,256 ist ein Atmungstestgerät gezeigt, das mit seiner Messung ebenfalls auf den Volumenstrom abzielt. Dort wird ein ohne Federbelastung in einem Zylinder angeordneter Kolben verwendet, der sich bei einem Atemzug einer einatmenden Person in dem Zylinder verschiebt und mit seiner Endlage den maximal während dieses Atemzuges erreichten Volumenstrom anzeigt. Weitere Geräte sind aus US 2015/0258370 A1, US 2011/0124470 A1, US 5,749,368, US 2014/0283831 A1 und EP 0937 438 A1 bekannt, die zumindest die Merkmale der Präambel des Anspruchs 1 offenbaren.

Es hat sich aber herausgestellt, dass die alleinige Betrachtung des erreichten maximalen Volumenstroms als Grundlage für die Bestimmung eines für eine mit Trockenpulverinhalation zu behandelnde Person passenden Trockenpulverinhalators oft nur ungenügende Ergebnisse liefert. Denn es gibt weitere wesentliche Parameter in der Atemleistung, die die Qualifizierung eines bestimmten Trockenpulverinhalators anhand des Atemwiderstandes ausmachen. Zu diesen Parametern zählt insbesondere der beim Einatmen erzeugte maximale Unterdruck, der, zusammen mit dem maximal erreichten Volumenstrom, eine Atemleistung bestimmt, und auch die maximal beim Einatmen erreichte Strömungsgeschwindigkeit.

Vor diesem Hintergrund ist es Aufgabe der Erfindung ein Messgerät und ein Verfahren anzugeben, mit dem das Atemvermögen einer Person (insbesondere eines Patienten in der Trockenpulverinhalation) einfach, kostengünstig und zuverlässig bestimmt bzw. klassifiziert werden kann.

Diese Aufgabe wird erfindungsgemäß gelöst durch ein Messgerät zum Bestimmen einer maximalen Inhalationsleistung einer Person, insbesondere für die Auswahl eines für die Person geeigneten Trockenpulverinhalationsgerätes, mit den Merkmalen des Anspruchs 1. Vorteilhafte Weiterbildungen sind in den Ansprüchen 2 bis 12 angegeben. Ein Verfahren, das die genannte Aufgabe löst, ist in Anspruch 13 bezeichnet.

Erfindungsgemäß wird also ein Messgerät zum Bestimmen einer maximalen Inhalationsleistung einer Person, insbesondere für die Auswahl eines für die Person geeigneten Trockenpulverinhalationsgerätes, mit

- einem Adapterstück zum strömungstechnischen Koppeln des Messgerätes mit dem Atemsystem der Person,

- einem mit dem Adapterstück verbundenen, zu einer Atemluftquelle offenen Strömungskanal, und

- einem in dem Strömungskanal angeordneten Strömungswiderstand,
  dadurch weitergebildet, dass in dem Strömungskanal zwischen dem Adapterstück und dem Strömungswiderstand ein Druckmesser zum Bestimmen eines bei einem Inspirationsvorgang der Person in dem Strömungskanal erzeugten Unterdruckes vorgesehen und angeordnet ist.

Mit einer solchen, insgesamt einfach aufzubauenden Vorrichtung lässt sich das Atemvermögen einer Person auf einer anderen und für die Auswahl eines passenden Trockenpulverinhalators verglichen mit den Parametern, die im Stand der Technik adressiert werden (nämlich der Volmenstrom), geeigneteren Basis ermitteln, nämlich anhand der Atemleistung in der Einatemspitze und der korrespondierenden Einatemgeschwindigkeit.

So lassen sich mit der erfindungsgemäßen Vorrichtung für einen gegebenen, ggf. wählbar vorgebbaren (vergl. Anspruch 3), Strömungswiderstand R der bei der Einatmung erzeugte Spitzenunterdruck $\Delta P_{max}$, die Spitzenströmungsgeschwindigkeit $U_{max}$ bestimmen, und damit auch die entsprechende Spitzenleistung nach der Umrechnung:

$$W_{max} = \Delta P_{max} \bullet \dot{U}_{max} \,.$$

Die Spitzenströmungsgeschwindigkeit lässt sich anhand des in der erfindungsgemäßen Vorrichtung bekannten, eingestellten Strömungswiderstandes (R) aus dem Zusammenhang

$$\dot{U}_{max} = \frac{1}{R} \bullet \Delta P_{max}$$

berechnen, bzw. es kann durch Einsetzen der zweiten in die erste oben stehende Gleichung die Spitzenleistung bestimmt werden zu

$$W_{max} = \frac{1}{R} \bullet \left(\Delta P_{max}\right)^2 \,.$$

Anhand dieser mit dem erfindungsgemäßen Messgerät über den maximalen Unterdruck $\Delta P_{max}$ und den Strömungswiderstand R bestimmten maximalen Atemleistung (Spitzenleistung, $W_{max}$) einer Person kann dann eine Auswahl eines für die Person passenden Trockenpulverinhalators vorgenommen werden. Hierfür kann z.B. auf dem erfindungsgemäßen Messgerät sogleich eine Anzeige der maximalen Atemleistung $W_{max}$ vorgesehen sein anstelle einer Anzeige des gemessenen maximalen Unterdrucks. In diese Anzeige ist dann der vorgegebenen Strömungswiderstand bereits eingeflossen, wobei, wenn mehrere Strömungswiderstände wählbar vorgebbar sind, entsprechend mehrere Anzeigen vorhanden sein können, die zu dem jeweiligen Strömungswiderstand passen. Auch kann für diesen Fall eine einzige Anzeige vorgesehen sein, die einen unter Einbeziehung des gewählten Strömungswiderstandes korrekt errechneten Wert der maximalen Atemleistung $W_{max}$ ausgibt, z.B. auf einem Display.

Die Anordnung des Druckmessers zwischen dem Strömungswiderstand und dem Adapterstück erlaubt insbesondere auch eine einfache mechanische Volumenstrommessung.

Das Adapterstück kann insbesondere als ein Mundstück ausgebildet sein, über das eine das Messgerät verwendende Person dieses wie das Mundstück eines Trockenpulverinhalators verwenden kann. So wird die Atemleistung beim Inhalieren besonders gut nachgebildet.

Eine besonders einfache Art, den vorgegebenen Strömungswiderstand zu realisieren, besteht darin, diesen als wenigstens eine eine vorgegebene Querschnittsöffnung aufweisende Blende im Strömungskanal, z.B. als wenigstens eine Einströmblende für einströmende Luft, zu bilden. Eine solche Blende, insbesondere Einströmblende, kann insbesondere an einer den Strömungskanal mit der Umgebungsluft verbindenden Gaseinströmöffnung des Messgerätes angeordnet sein.

Wenn der Strömungswiderstand aus vorgegeben einstellbaren Widerständen variabel gestaltet werden soll, so kann dies bei einer Realisierung der Strömungswiederstände als Blenden, insbesondere Einströmblenden, dadurch geschehen, dass wenigstens zwei oder auch mehr Blenden, insbesondere Einströmblenden, mit unterschiedlichen Querschnittsöffnungen vorgesehen sind und dass ein Umschaltelement vorgesehen ist, mit welchem wahlweise wenigstens eine der zwei oder mehr Blenden, insbesondere Einströmblenden, freigegeben, verbleibende, nicht freizugebende Blenden, insbesondere Einströmblenden, dagegen verschlossen werden können. Diese Art eines Umschaltens zwischen verschiedenen Blenden bzw. Einströmblenden in dem erfindungsgemäßen Messgerät stellt eine einfache und kostengünstige Umsetzung einer wählbaren Vorgabemöglichkeit verschiedener, vorgegebener Strömungswiderstände dar. Dabei kann ein Strömungswiderstand auch durch zwei oder mehr freigegebene Blenden, insbesondere Einströmblenden, gebildet werden.

Umgesetzt werden kann diese Gestaltungsvariante insbesondere, indem die zwei oder mehr Blenden, insbesondere Einströmblenden, auf einem im Querschnitt kreisförmigen Abschnitt des Messgerätes in Umfangsrichtung verteilt angeordnet sind und indem weiterhin das Umschaltelement eine auf dem im Querschnitt kreisförmigen Abschnitt angeordnete Hülse ist, die wenigstens eine Öffnung aufweist, die in ihrem Querschnitt größer ist als oder ebenso groß ist wie die die größte Querschnittsöffnung aufweisende Einströmblende, wobei der kreisförmige Abschnitt mit den zwei oder mehr Blenden, insbesondere Einströmblenden und die Hülse relativ zueinander verdrehbar sind, wobei durch Verdrehen des kreisförmigen Abschnittes relativ zu der Hülse die wenigstens eine Öffnung in der Hülse wahlweise mit wenigstens einer der Blenden, insbesondere Einströmblenden, so in Überdeckung gebracht werden kann, dass diese Blende(n), bzw. Einströmblende(n) vollständig offen liegt/liegen, während eine oder mehrere ausgewählte weitere Blenden, bzw. Einströmblenden durch die Hülse verdeckt und verschlossen werden können.

Der Druckmesser in dem erfindungsgemäßen Messgerät kann besonders einfach als ein in einem an einem ersten Ende mit dem Strömungskanal verbundenen Zylinder mit einem darin entgegen einer Federkraft in Richtung des ersten Endes verlagerbar angeordneten Kolben gebildet sein, wobei die Fortbewegung des Kolbens in Richtung des ersten Endes während eines Inspirationsvorganges der Person ein Maß für den Unterdruck ist. Diese Bauweise ist robust und dabei einfach und kostengünstig aus wenigen Teilen herzustellen. Sie kann insbesondere sehr kompakt in ein insbesondere stiftförmiges Messgerät integriert werden. Insbesondere ist der Zylinder an seinem dem ersten Ende gegenüberliegenden Ende zum Umgebungsdruck hin geöffnet, in dem zwischen den Enden liegenden Bereich vollständig verschlossen.

Bei einer wie vorstehend beschriebenen Bauweise des Druckmessers kann die Anzeige des Unterdrucks bzw. der daraus nach obigen Rechenvorschriften abgeleiteten maximalen Atemleistung auf einfache Weise durch ein in oder an dem Zylinder angeordnetes, mit dem Kolben nicht verbundenes, von dem Kolben bei einer Verlagerung des Kolbens in Richtung des ersten Endes mitgeführtes Mitnahmeteil realisiert werden, das bei einer Rückverlagerung des Kolbens weg von dem ersten Ende in der aufgrund einer Verlagerung des Kolbens in Richtung des ersten Endes erreichten Position verbleibt. Die erreichte Position kann dann im Zusammenwirken mit einer an dem Zylinder aufgebrachten Skala zur Anzeige des erreichten Unterdrucks bzw. eines daraus abgeleiteten Parameters, insbesondere der maximalen Atemleistung oder Spitzenleistung $W_{max}$ in quantitativer und/oder qualitativer Weise dienen. Dabei kann z.B. ein Wert für den Unterdruck oder ein Wert für die maximale Atemleistung angezeigt werden. Es können auch mehrere Skalen parallel zueinander angeordnet und z.B. farblich unterschieden sein, wenn es mehrere unterschiedliche, wählbare vorgegebene Strömungswiderstände in dem Messgerät gibt. Für eine qualitative Anzeige kann z.B. die Skala in verschiedene Bereiche, z.B. drei Bereiche, die mit entsprechenden Symbolen, z.B. A, B, C usw., oder anderen Kennzeichnungen, wie Farben, unterscheidbar gekennzeichnet sind, unterteilt sein, wobei die jeweiligen Symbole passenden Typengruppen von Trockeninhalatoren mit für die Person, die das Messgerät verwendet, geeigneten Strömungswiderständen zugeordnet sind.

Das Messgerät kann in einer einfachen Bauform insgesamt geradlinig und im Wesentlichen stift- bzw. rohrförmig gebildet sein. Dies erlaubt eine einfache Herstellung, z.B. aus spritzgegossenen Kunststoffteilen.

So kann das Messgerät z.B. als Einwegartikel konzipiert und bspw. Trockeninhalatoren als Testgerät mit beigegeben oder einem Behandler, der den für eine Person passenden Trockeninhalator auswählt, kostengünstig zur Verfügung gestellt werden. Eine Herstellung des Messgeräts aus Kunststoff wird allgemein wegen der geringen Kosten und der einfachen und kostengünstigen Verarbeitbarkeit dieses Materials bevorzugt werden.

Gegenstand der Erfindung und Lösung der Aufgabe ist weiterhin ein Verfahren zum Bestimmen einer maximalen Inhalationsleistung einer Person, bei dem die Person durch einen mit einem vorgegebenen Strömungswiderstand R versehenen Strömungskanal einatmet und während des Einatemvorganges ein an einer ausgehend von dem Strömungswiderstand in Richtung des Atemtraktes der Person gelegenen Stelle ein maximal auftretender Unterdruck $\Delta P_{max}$ bestimmt wird, wobei aus dem aufgetretenen Unterdruck und dem Strömungswiderstand nach der Vorschrift

$$W_{max} = \frac{1}{R} \bullet \left( \Delta P_{max} \right)^2$$

auf eine maximale Atemleistung $W_{max}$ geschlossen wird.

Weitere Vorteile und Merkmale der Erfindung ergeben sich aus der nachfolgenden Beschreibung eines Ausführungsbeispiels anhand der beigefügten Figuren. Dabei zeigen:

Fig. 1    eine dreidimensionale Schrägansicht eines möglichen Ausführungsbeispiels eines erfindungsgemäßen Messgeräts zum Bestimmen einer maximalen Inhalationsleistung einer Person;

Fig. 2    ein schematisches Schaltbild zur Verdeutlichung des Strömungskanals und der Anordnung der wesentlichen Funktionselemente des erfindungsgemäßen Messgerätes in dem Strömungskanal und

Fig. 3    eine Darstellung der einzelnen Teile des in Fig. 1 gezeigten Ausführungsbeispiels eines erfindungsgemäßen Messgerätes.

In den Figuren ist ein Ausführungsbeispiel für ein erfindungsgemäßes Messgerät zum Bestimmen einer maximalen Inhalationsleistung einer Person gezeigt und mit den wesentlichen Komponenten dargestellt. In den Figuren sind gleiche Elemente mit gleichen Bezugszeichen versehen.

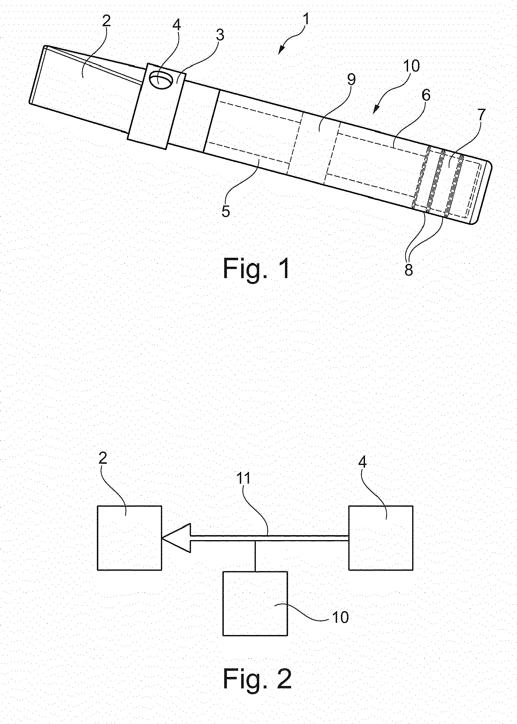

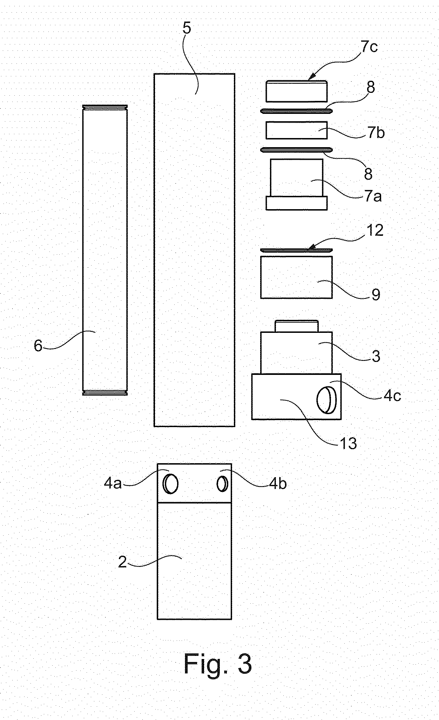

In Fig. 1 ist eine dreidimensionale Schrägansicht eines möglichen Ausführungsbeispiels eines erfindungsgemäßen Messgerätes 1 zum Bestimmen einer maximalen Inhalationsleistung einer Person gezeigt. Das Messgerät 1 ist im Wesentlichen stift- bzw. rohrförmig geformt und verfügt über ein Adapterstück in Form eines Mundstückes 2, welches von einer das Messgerät 1 benutzenden Personen mit den Lippen umschlossen und so mit ihrem Atemweg gekoppelt werden kann. Das Mundstück 2 ist in einem Anschlussstück 3 lösbar aufgenommen, insbesondere in einem hülsenförmigen Abschnitt 13 (vergleiche Fig. 3) des Anschlussstücks 3 eingesetzt. Im Zusammenspiel mit dem Anschlussstück 3 bildet das Mundstück 2 eine Einströmblende 4 aus, durch die bei einem Einatemvorgang durch das Mundstück 2 Umgebungsluft in das Messgerät 3 eingesaugt werden kann und durch die Einströmblende in den zwischen der Einstrahlungsblende 4 und der Öffnung des Mundstücks 2 gebildeten Strömungskanal einströmt.

Das Anschlussstück 3 sitzt an einer dem Abschnitt 13 gegenüberliegenden Seite auf einem offenen ersten Ende eines Zylinderrohrs 5 auf. Dieses Zylinderrohr 5 ist in dem gezeigten Ausführungsbeispiel aus einem transparenten Kunststoff, z.B. aus PMMA, gebildet. An seinem dem mit dem Anschlussstück 3 verbundenen ersten Ende gegenüberliegenden zweiten Ende ist das Zylinderrohr 5 gegenüber dem Umgebungsdruck offen. Umfangsseitig ist das Zylinderrohr 5 verschlossen. In dem Zylinderrohr 5 ist ein Kolben 7 angeordnet, der mit Dichtlippen 8 gegen eine Innenwand des Zylinderrohres 5 abgedichtet. An dem zweiten Ende des Zylinderrohrs 5 ist ein Anschlag für den Kolben 7 gebildet. Der Kolben 7 ist durch die Kraft einer in dem Zylinderrohr 5 in dessen Längsrichtung wirkend angeordneten Feder 6 in Richtung des zweiten Endes des Zylinder 5 vorgespannt und ruht an dem zweiten Ende in einer Ausgangsstellung. Weiterhin ist ein in dem Zylinderrohr 5 angeordneter Mitnahmering 9 zu erkennen, der in der Fig. 1 in etwa mittig des Zylinderrohres 5 gezeigt ist. Tatsächlich sitzt in einem Ausgangszustand des Messgerätes 1 der Mitnahmering 9 mit einer in Fig. 1 rechts gelegenen Anschlagfläche an einer in Fig. 1 links gezeigten Mitnahmefläche des Kolbens 7, also zum zweiten Ende des Zylinderrohres 5 hin verschoben.

Die Elemente Zylinderrohr 5, Feder 6, Kolben 7 mit Dichtlippen 8 und Mitnahmering 9 bilden zusammen einen Druckmesser 10 zum Messen eines beim Einatmen einer das Messgerät 1 benutzenden Personen entstehenden (maximalen) Unterdrucks.

In Fig. 2 ist der Strömungsweg der beim Verwenden des erfindungsgemäßen Messgerätes 1 durch dieses strömenden Atemluft bzw. der in dem Messgerät 1 ausgebildete Strömungskanal 11 schematisch veranschaulicht zusammen mit der Lage der Elemente Mundstück 2, Einströmblende 4 und Druckmesser 10 relativ zueinander und in Bezug auf den Strömungskanal 11. Zu erkennen ist hier der wesentliche Aspekt, dass der Druckmesser 10 in dem Strömungskanal 11 zwischen der Einströmblende 4 und dem Mundstück 2 angeordnet ist. Diese Anordnung erlaubt es, durch die festgelegte, als Strömungswiderstand dienende Einströmblende 4 einerseits auf die Strömungsgeschwindigkeit bei einem Einatemvorgang einer das erfindungsgemäße Messgerät 1 benutzenden Personen zu schließen, andererseits dabei zugleich auch den beim Einatmen erzeugten Unterdruck zu bestimmen.

In Fig. 3 sind noch einmal die Einzelteile des in Fig. 1 dargestellten erfindungsgemäßen Messgerätes 1 aufgezeigt. Zu erkennen ist hier zunächst, dass das Mundstück 2 mit wenigstens zwei Blendenöffnungen 4a, 4b versehen ist, die auf einem Umfangsabschnitt des Mundstücks 2 voneinander beanstandet und verteilt angeordnet sind. Weiterhin zu erkennen ist, dass an dem Anschlussstück 3 eine Öffnung Ziffer 4c angebracht ist, deren Öffnungsweite größer ist als die Öffnungsweiten der Blendenöffnungen 4a, 4b an dem Mundstück 2. Das Mundstück 2 kann nun relativ zu dem Anschlussstück 3 in der Umfangsrichtung verdreht werden, so dass eine der Blendenöffnungen 4a bzw. 4b mit der Öffnung 4c in Überdeckung gebracht und dadurch eine Einströmblende mit vorgegebenem Strömungswiderstand gebildet werden kann. Selbstverständlich können die Blendenöffnungen 4a, 4b ebenso gut an dem Anschlussstück 3 und kann die Öffnung 4 an dem Mundstück ausgebildet sein. Auch andere Lösungen sind denkbar, z.B. eine Ausbildung der Blendenöffnungen 4a, 4b an Mundstück 2 oder Anschlussstück 3 und vorsehen eines auf den Abschnitt mit den Blendenöffnungen 4a, 4b aufgebrachten Ringes mit der Öffnung 4, der durch Verdrehen eine der Blendenöffnungen 4a, 4b freigibt, die restlichen Blendenöffnungen aber verdeckt.

Zu erkennen ist weiterhin, dass der Kolben 7 mehrteilig aufgebaut ist, mit einem Kolbenkorpus 7a, auf dem eine erste Dichtlippe 8, ein Kolbenring 7b, eine zweite Dichtlippe 8 und ein Kolbendeckel 7c aufgebracht sind. Ebenfalls zu erkennen ist der Mitnahmering 9, wobei zwischen dem Mitnahmering 9 und dem Kolben 7, genauer dem Kolbenkorpus 7a, eine weitere Dichtlippe 12 angeordnet ist. Diese Dichtlippe 12 ist an dem Mitnahmering 9 festgelegt und sorgt insbesondere dafür, dass der Mitnahmering 9 in einer bei einem Verschieben des Kolbens 7 durch Erzeugen eines Unterdrucks in dem Zylinderrohr 5 beim Einatmen erfolgenden Verlagern des Mitnahmerings 9 nicht aus der einmal eingenommenen Position herausfällt, wenn der Kolben 7 sich, getrieben durch die Rückstellkraft der Feder 6, wieder zurück in seine Ausgangsposition bewegt.

Das Messgerät 1 ist mit seinen Komponenten insbesondere aus kostengünstigen Materialien hergestellt. So sind insbesondere die Elemente Mundstück 2, Anschlussstück 3, Zylinderrohr 5, Kolben 7 und Mitnahmering 9 aus Kunststoff gebildet, insbesondere als Spritzgussteile geformt. Lediglich die Feder 6 ist aus einem Metall, zum Beispiel einem Stahl, hergestellt. Die Dichtlippen 8, 12 sind aus einem weichen Kunststoffmaterial, zum Beispiel Silikon, gebildet. So ist das erfindungsgemäße Messgerät 1 gemäß diesem Ausführungsbeispiel als kostengünstig herzustellendes Einweggerät konzipiert, das zum Beispiel verschiedenen Trockenpulverinhalatoren beigegeben oder Anwendern solcher Geräte überlassen werden kann.

Gebrauch des in dem Ausführungsbeispiel gezeigten, erfindungsgemäßen Messgerätes 1 und seine Funktion werden nachfolgend beschrieben. Für das Messen ihrer maximalen Inhalationsleistung nimmt eine das Messgerät 1 nutzende Person das Mundstück 2 zwischen die Lippen und atmet durch den Mund ein. Dabei wurde zuvor durch entsprechendes in Deckung Bringen der Öffnung 4 mit einer der Blendenöffnungen 4a bzw. 4b eine Einströmblende 4 mit geeigneter Durchtrittsöffnung und darüber ein passender Strömungswiderstand eingestellt. Beim Einatmen strömt nun Luft von außerhalb des Messgerätes 1 durch die Einströmblende 4 in den Strömungskanal 11 des Messgerätes 1 ein und streicht dabei an dem ersten, offenen Ende des Zylinderrohrs 5 vorbei. Der durch den Atemhub in dem Strömungskanal 11 erzeugte Unterdruck besorgt nun, dass in dem Zylinderrohr 5 der Kolben 7 entgegen der Kraft der Feder 6 in Richtung des ersten Endes verlagert wird. Dabei nimmt der Kolben 7 den Mitnahmering 9 mit bis in eine maximal zum ersten Ende des Zylinderrohrs 5 hin reichende Position, die der Kolben 7 bei einem maximalen Unterdruck erreicht. Endet der Atemzug der Person, so wird der Kolben 7 durch die Kraft der Feder 6 wieder zurück in seine Ausgangsposition am zweiten Ende des Zylinderrohrs 5 gedrückt. Der Mitnahmering 9 verbleibt in der Position, bis zu der der Kolben 7 diesen mitgenommen und im Zylinderrohr 5 verschoben hat. Diese eingenommene Position ist nun ein Maß für den maximal bei dem Atemzug der Person, die das Messgerät 1 gebraucht hat, erreichten Unterdruck.

Da über die vorgegebene Einstellung der Einströmblende 4 und mit dieser des Strömungswiderstandes eine Beziehung zur Strömungsgeschwindigkeit geschaffen ist, werden auf diese Weise also Strömungsgeschwindigkeit und maximaler Unterdruck korreliert, kann durch die erreichte Stellung des Mitnahmerings 9 nach der vorstehend in der allgemeinen Beschreibung hergeleiteten Gleichung und Formel auf eine maximale Atemleistung der das Messgerät 1 verwendenden Person geschlossen werden. Um diese maximalen Leistung möglichst leicht ablesen zu können, können - und sind dies bevorzugt - an dem Zylinderrohr 5 skalenartige Markierungen angeordnet sein, die entsprechende Werte qualitativ oder quantitativ, zum Beispiel durch unterschiedlich eingefärbte Bereiche, ablesen lassen.

Auch die vorstehende Beschreibung des Ausführungsbeispiels belegt noch einmal klar, dass mit der Erfindung ein einfach aufgebautes, kostengünstig herstellbares und einfach zu bedienendes Messgerät zum Bestimmen einer maximalen Inhalationsleistung einer Person geschaffen worden ist, das durch eine Bestimmung einer maximalen Atemleistung einen besonders aussagekräftigen Parameter für eine spätere Zuteilung eines für die Person passenden Trockenpulverinhalators erlaubt.

**Bezugszeichenliste**

| 1 | Messgerät |
| 2 | Mundstück |
| 3 | Anschlussstück |
| 4 | Einströmblende |
| 4a | Blendenöffnung |
| 4b | Blendenöffnung |
| 4c | Öffnung |
| 5 | Zylinderrohr |
| 6 | Feder |
| 7 | Kolben |
| 7a | Kolbenkorpus |
| 7b | Kolbenring |
| 7c | Kolbendeckel |
| 8 | Dichtlippe |
| 9 | Mitnahmering |
| 10 | Druckmesser |
| 11 | Strömungskanal |
| 12 | Dichtlippe |
| 13 | Abschnitt |

**Patentansprüche**

1. Messgerät (1) zum Bestimmen einer maximalen Inhalationsleistung einer Person, insbesondere für die Auswahl eines für die Person geeigneten Trockenpulverinhalationsgerätes, mit

   - einem Adapterstück (2) zum strömungstechnischen Koppeln des Messgerätes (1) mit dem Atemsystem der Person,
   - einem mit dem Adapterstück (2) verbundenen, zu einer Atemluftquelle offenen Strömungskanal (11), und
   - einem in dem Strömungskanal (11) angeordneten Strömungswiderstand (4), mit einem in dem Strömungskanal (11) zwischen dem Adapterstück (2) und dem Strömungswiderstand angeordneten Druckmesser (10) zum Bestimmen eines bei einem Inspirationsvorgang der Person in dem Strömungskanal (11) erzeugten Unterdruckes, **dadurch gekennzeichnet, dass** der Druckmesser (10) durch einen in einem an einem ersten Ende mit dem Strömungskanal (11) verbundenen Zylinder (5) mit einem darin entgegen einer Federkraft in Richtung des ersten Endes verlagerbar angeordneten Kolben (7) gebildet ist, wobei die Fortbewegung des Kolbens (7) in Richtung des ersten Endes während eines Inspirationsvorganges der Person ein Maß für den Unterdruck ist.

2. Messgerät (1) nach Anspruch 1, **dadurch gekennzeichnet, dass** das Adapterstück (2) als Mundstück ausgebildet ist.

3. Messgerät (1) nach einem der vorhergehenden Ansprüche, **dadurch gekennzeichnet, dass** der Strömungswiderstand variabel einstellbar ist.

4. Messgerät (1) nach einem der vorhergehenden Ansprüche, **dadurch gekennzeichnet, dass** der Strömungswiderstand durch wenigstens eine eine vorgegebene Querschnittsöffnung aufweisende Einströmblende (4) gebildet ist.

5. Messgerät (1) nach Anspruch 4, **dadurch gekennzeichnet, dass** die wenigstens eine Einströmblende (4) an einer den Strömungskanal (11) mit der Umgebungsluft verbindenden Gaseinströmöffnung vorgesehen ist.

6. Messgerät (1) nach den Ansprüchen 3 und 4 oder 3 und 5, **dadurch gekennzeichnet, dass** wenigstens zwei Einströmblenden (4a, 4b) mit unterschiedlichen Querschnittsöffnungen vorgesehen sind und dass ein Umschaltelement vorgesehen ist, mit welchem wahlweise wenigstens eine der wenigstens zwei Einströmblenden (4a, 4b) freigegeben, verbleibende, nicht freizugebende Einströmblenden (4a, 4b) dagegen verschlossen werden können.

7. Messgerät (1) nach Anspruch 6, **dadurch gekennzeichnet, dass** die wenigstens zwei Einströmblenden (4a, 4b) auf einem im Querschnitt kreisförmigen Abschnitt des Messgerätes (1) in Umfangsrichtung verteilt angeordnet sind und dass das Umschaltelement eine auf dem im Querschnitt kreisförmigen Abschnitt angeordnete Hülse (13) ist, die wenigstens eine Öffnung (4c) aufweist, die in ihrem Querschnitt größer ist als oder ebenso groß ist wie die die größte Querschnittsöffnung aufweisende Einströmblende (4a, 4b), wobei der kreisförmige Abschnitt mit den wenigstens zwei Einströmblenden (4a, 4b) und die Hülse (13) relativ zueinander verdrehbar sind, wobei durch Verdrehen des kreisförmigen Abschnittes relativ zu der Hülse (13) die wenigstens eine Öffnung (4c) in der Hülse (13) wahlweise mit einer der Einströmblenden (4a, 4b) so in Überdeckung gebracht werden kann, dass diese Einströmblende (4a, 4b) vollständig offen liegt, während ausgewählte weitere Einströmblenden (4a, 4b) durch die Hülse (13) verdeckt und verschlossen werden können.

8. Messgerät (1) nach einem der vorhergehenden Ansprüche, **gekennzeichnet durch** ein in oder an dem Zylinder (5) angeordnetes, mit dem Kolben (7) nicht verbundenes, von dem Kolben (7) bei einer Verlagerung des Kolbens (7) in Richtung des ersten Endes mitgeführtes Mitnahmeteil (9), das bei einer Rückverlagerung des Kolbens (7) weg von dem ersten Ende in der aufgrund einer Verlagerung des Kolbens (7) in Richtung des ersten Endes erreichten Position verbleibt.

9. Messgerät (1) nach Anspruch 8 **gekennzeichnet**

**durch** eine Skala an dem Zylinder (5), auf der das Mitnahmeteil (9) mit einer eingenommenen Position den erreichten Unterdruck bzw. einen daraus abgeleiteten Parameter in quantitativer und/oder qualitativer Weise anzeigt.

10. Messgerät (1) nach einem der vorhergehenden Ansprüche, **dadurch gekennzeichnet, dass** dieses insgesamt geradlinig und im Wesentlichen stift- oder rohrförmig gebildet ist.

11. Messgerät (1) nach einem der vorhergehenden Ansprüche, **dadurch gekennzeichnet, dass** es, zumindest überwiegend, aus Kunststoff besteht.

12. Verfahren zum Bestimmen einer maximalen Inhalationsleistung einer Person, wobei die Person durch einen mit einem vorgegebenen Strömungswiderstand R versehenen Strömungskanal einatmet und während des Einatemvorganges ein an einer ausgehend von dem Strömungswiderstand in Richtung des Atemtraktes der Person gelegenen Stelle ein maximal auftretender Unterdruck $\Delta P_{max}$ bestimmt wird, wobei aus dem aufgetretenen Unterdruck und dem Strömungswiderstand nach der Vorschrift

$$W_{max} = \frac{1}{R} \bullet \left( \Delta P_{max} \right)^2$$

auf eine maximale Atemleistung $W_{max}$ geschlossen wird, wobei ein Messgerät nach einem der vorstehenden Ansprüche Verwendung findet.

## Claims

1. A measuring device (1) for determining a maximum inhalation performance of a person, in particular for selecting a dry powder inhaler suitable for the person, with

     - an adapter piece (2) for fluidic coupling of the measuring device (1) with the breathing system of the person,
     - a flow channel (11) connected to the adapter piece (2) and open to a breathing air source, and
     - a flow resistor (4) arranged in the flow channel (11), comprising a pressure gauge (10) arranged in the flow channel (11) between the adapter piece (2) and the flow resistor for determining a negative pressure generated in the flow channel (11) during an inspiration process of the person,
     **characterised in that** the pressure gauge (10) is formed by a cylinder (5) connected at a first end to the flow channel (11) and having a piston (7) arranged therein so as to be displaceable against a spring force in the direction of the first end, the advance of the piston (7) in the direction of the first end during an inspiratory process of the person being a measure of the suppression.

2. The measuring device (1) according to claim 1, **characterised in that** the adapter piece (2) is formed as a mouthpiece.

3. The measuring device (1) according to one of the preceding claims, **characterised in that** the flow resistance is variably adjustable.

4. The measuring device (1) according to one of the preceding claims, **characterised in that** the flow resistance is formed by at least one inflow orifice (4) having a predetermined cross-sectional opening.

5. The measuring device (1) according to claim 4, **characterised in that** said at least one inflow orifice (4) is provided at a gas inflow opening connecting the flow channel (11) to the ambient air.

6. The measuring device (1) according to claims 3 and 4 or 3 and 5, **characterised in that** at least two inflow orifices (4a, 4b) with different cross-sectional openings are provided and that a switch-over element is provided with which optionally at least one of said at least two inflow orifices (4a, 4b) can be released, while remaining inflow orifices (4a, 4b) which cannot be released can be closed.

7. The measuring device (1) according to claim 6, **characterised in that** said at least two inflow membranes (4a, 4b) are arranged on a section of the measuring device (1) which is circular in cross-section and distributed in the circumferential direction, and **in that** the change-over element is a sleeve (13) arranged on the section which is circular in cross-section, which sleeve (13) has at least one opening (4c) which is larger in cross-section than or is as large as the inflow membrane (4a, 4b) having the largest cross-sectional opening, wherein the circular section with the at least two inflow orifices (4a, 4b) and the sleeve (13) are rotatable relative to one another, wherein by rotating the circular section relative to the sleeve (13) said at least one opening (4c) in the sleeve (13) is selectively connected to one of the inflow orifices (4a, 4b), 4b) can be overlapped in such a way that this inflow membrane (4a, 4b) is completely open, while selected further inflow membranes (4a, 4b) can be covered and closed by the sleeve (13).

8. The measuring device (1) according to any of the preceding claims, **characterised by** a driving part (9) which is arranged in or on the cylinder (5), is not connected to the piston (7), is driven by the piston (7) when the piston (7) is displaced in the direction of the first end, and which, when the piston (7) is displaced backwards away from the first end, is displaced in the direction of the second end in the direction of the first end in the flow direction caused by the piston (7) in the direction of the first end.

9. The measuring device (1) according to claim 8 **characterised by** a scale on the cylinder (5) on which the driving part (9) with an assumed position indicates in a quantitative and/or qualitative manner the achieved suppression or a parameter derived therefrom.

10. The measuring device (1) according to one of the preceding claims, **characterised in that** it is generally rectilinear and substantially pin- or tubular-shaped.

11. The measuring device (1) according to one of the preceding claims, **characterised in that** it consists, at least predominantly, of plastic.

12. A method for determining a maximum inhalation performance of a person, wherein the person inhales through a flow channel provided with a predetermined flow resistance R and during the inhalation process a maximum occurring suppression APmax is determined at a point located starting from the flow resistance in the direction of the respiratory tract of the person, wherein from the occurred suppression and the flow resistance according to the following equation

$$W_{max} = \frac{1}{R} \bullet \left( \Delta P_{max} \right)^2$$

is concluded to a maximum breathing capacity Wmax, using a measuring device according to one of the above claims.

**Revendications**

1. Dispositif de mesure (1) pour déterminer une puissance d'inhalation maximale d'une personne, en particulier pour le choix d'un inhalateur de poudre sèche adapté à la personne, avec

- un adaptateur (2) pour le couplage fluidique de l'appareil de mesure (1) avec le système respiratoire de la personne,
- un canal d'écoulement (11) relié à l'adaptateur (2) et débouchant sur une source d'air respirable, et
- une résistance d'écoulement (4) disposée dans le canal d'écoulement (11), comprenant un manomètre (10) disposé dans le canal d'écoulement (11) entre l'adaptateur (2) et la résistance d'écoulement pour déterminer une pression négative générée dans le canal d'écoulement (11) pendant un processus d'inspiration de la personne, **caractérisé en ce que** le manomètre (10) est formé par un cylindre (5) relié à une première extrémité au canal d'écoulement (11) et muni d'un piston (7) disposé dans celui-ci de manière à pouvoir être déplacé contre une force de ressort dans la direction de la première extrémité, l'avance du piston (7) dans la direction de la première extrémité pendant un processus inspiratoire de la personne étant une mesure de la dépression.

2. Dispositif de mesure (1) selon la revendication 1, **caractérisé en ce que** l'adaptateur (2) est sous forme d'embout.

3. Dispositif de mesure (1) selon l'une des revendications précédentes, **caractérisé en ce que** la résistance à l'écoulement est réglable de manière variable.

4. Dispositif de mesure (1) selon l'une des revendications précédentes, **caractérisé en ce que** la résistance à l'écoulement est constitué d'au moins un orifice d'entrée (4) munie d'une ouverture de section transversale prédéterminée.

5. Dispositif de mesure (1) selon la revendication 4, **caractérisé en ce que** ledit moins un orifice d'entrée (4) est prévu à une ouverture d'entrée de gaz reliant le canal d'écoulement (11) à l'air ambiant.

6. Dispositif de mesure (1) selon les revendications 3 et 4 ou 3 et 5, **caractérisé en ce qu'**il est prévu au moins deux orifices d'entrée (4a, 4b) avec des ouvertures de section transversale différentes et **en ce qu'**il est prévu un élément de commutation avec lequel, au choix, au moins l'un des au moins deux orifices d'entrée (4a, 4b) peut être déverrouillé, tandis que les autres orifices d'entrée (4a, 4b) qui ne peuvent pas être libérés peuvent être fermés.

7. Dispositif de mesure (1) selon la revendication 6, **caractérisé en ce que** lesdits au moins deux membranes d'entrée (4a, 4b) sont disposés sur une section du dispositif de mesure (1) de section transversale circulaire et répartis dans la direction périphérique, et **en ce que** l'élément de commutation est un manchon (13) disposé sur la section transversale circulaire, lequel manchon (13) présente au moins une ouverture (4c) de section transversale plus grande que la membrane d'entrée (4a, 4b) présentant la plus grande ouverture en section transversale ou qui est aussi grande que celui-ci, dans lequel la section circulaire avec les au moins deux orifices d'entrée (4a, 4b) et le manchon (13) peuvent tourner l'un par rapport à l'autre, dans lequel, par rotation de la section circulaire par rapport au manchon (13), l'au moins une ouverture (4c) dans le manchon (13) est reliée de manière sélective à l'un des orifices d'entrée (4a, 4b), 4b) peuvent se chevaucher de telle sorte que cette membrane d'entrée (4a, 4b) est complètement ouverte, tandis que d'autres membranes d'entrée (4a, 4b) sélectionnées peuvent être recouvertes et fermées par le manchon (13).

8. Dispositif de mesure (1) selon l'une des revendications précédentes, **caractérisé par** une partie d'entraînement (9) disposée dans ou sur le cylindre (5), qui n'est pas reliée au piston (7), qui est entraînée par le piston (7) lorsque le piston (7) est déplacé en direction de la première extrémité, et qui, lorsque le piston (7) est déplacé vers l'arrière en s'éloignant de la première extrémité, est déplacée en direction de la deuxième extrémité en direction de la première extrémité dans la direction d'écoulement provoquée par le piston (7) en direction de la première extrémité.

9. Dispositif de mesure (1) selon la revendication 8, **caractérisé par** une graduation sur le cylindre (5), sur laquelle la partie d'entraînement (9) avec une position prise indique de manière quantitative et/ou qualitative la dépression obtenue ou un paramètre dérivé de celle-ci.

10. Dispositif de mesure (1) selon l'une des revendications précédentes, **caractérisé en ce qu'**il est généralement rectiligne et sensiblement en forme de broche ou de tube.

11. Dispositif de mesure (1) selon l'une des revendications précédentes, **caractérisé en ce qu'**il est constitué, au moins principalement, de matière plastique.

12. Procédé pour déterminer une puissance d'inhalation maximale d'une personne, dans lequel la personne inhale à travers un canal d'écoulement pourvu d'une résistance à l'écoulement R prédéterminée et, pendant le processus d'inhalation, une suppression APmax maximale se produisant est déterminée à un point situé en partant de la résistance à l'écoulement dans la direction des voies respiratoires de la personne, dans lequel, à partir de la suppression produite et de la résistance à l'écoulement selon la formule

$$W_{\max} = \frac{1}{R} \bullet \left( \Delta P_{\max} \right)^2$$

est conclu à une capacité respiratoire maximale Wmax, en utilisant un appareil de mesure selon l'une des revendications ci-dessus.

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 937438 A1
- US 4693256 A
- US 20150258370 A1
- US 20110124470 A1
- US 5749368 A
- US 20140283831 A1
- EP 0937438 A1